# EUROPÄISCHE PATENTANMELDUNG

(11) **EP 2 070 942 A1**
(43) Veröffentlichungstag der Anmeldung: **17.06.2009**
(21) Anmeldenummer: 07076136.6
(22) Anmeldetag: 13.12.2007
(51) Int. Cl.: C07J 1/00, C07J 41/00

(54) **Verfahren zur Aromatisierung von 19-Nor-androst-4-en-3-onen zu Estra-1,3,5(10)-trienen**

(71) Anmelder: Bayer Schering Pharma Aktiengesellschaft, 13353 Berlin (DE); AstraZeneca AB, 151 85 Södertälje (SE)
(72) Erfinder: Sander, Michael, 50226 Frechen (Königsdorf) (DE); Gries, Jörg, 42781 Haan (DE); Schütz, Armin, 13465 Berlin (DE)

(57) **Zusammenfassung**

Die vorliegende Erfindung betrifft ein Verfahren zur Aromatisierung von 19-Norandrost-4-en-3-onen (Formel (II)) zu Estra-1,3,5(10)-trienen ((Formel I))

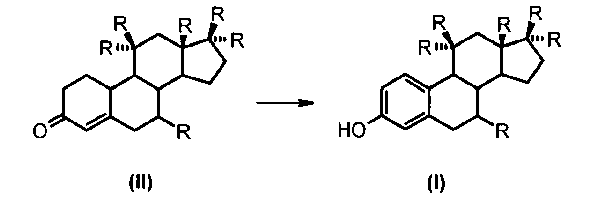

## Beschreibung

Die vorliegende Erfindung betrifft ein Verfahren zur Aromatisierung von 19-Norandrost-4-en-3-onen (Formel (II)) zu Estra-1,3,5(10)-trienen ((Formel I)) gemäß Schema 1 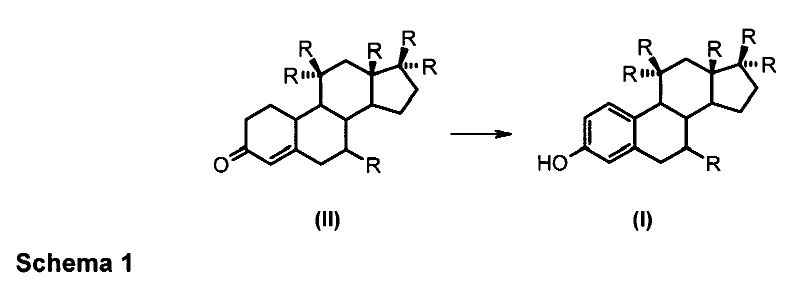 wobei in Formel (II) und (I) alle R unabhängig voneinander für chemisch stabile beliebige Reste stehen.

Der Erfindung liegen folgende Definitionen zu Grunde:

### Cₙ-Alkyl:

Monovalenter, geradkettiger oder verzweigter, gesättigter Kohlenwasserstoffrest mit n Kohlenstoffatomen.

### Cₙ₋Alkylen:

divalenter, geradkettiger oder verzweigter, gesättigter Kohlenwasserstoffrest mit n Kohlenstoffatomen.

### Cₙ-Alkenyl:

monovalenter, geradkettiger oder verzweigter Kohlenwasserstoffrest mit n Kohlenstoffatomen und mindestens einer Doppelbindung.

### Cₙ-Alkinyl:

Monovalenter, geradkettiger oder verzweigter Kohlenwasserstoffrest mit n Kohlenstoffatomen und mindestens einer Dreifachbindung.

### Cₙ-Cycloalken

cyclischer einfach ungesättigter Kohlenwasserstoffring mit n Kohlenstoffatomen

### Cₙ-Alkoxy:

Geradkettiger oder verzweigter Cₙ-Alkyletherrest der Formel -OR mit R=Cₙ-Alkyl.

### Cₙ-Alkenoxy:

Geradkettiger oder verzweigter Cₙ-Alkenyletherrest der Formel -OR mit R=Cₙ-Alkenyl.

### Cₙ-Acyloxy:

Cₙ-Acyloxy steht für einen linearen oder verzweigten Cₙ-Alkylesterrest der Formel -O-C(O)-Cₙ-Alkyl.
In der Regel ist n gleich 1 bis 6, bevorzugt 1 bis 4, und besonders bevorzugt 1 bis 3. Beispielhaft und vorzugsweise seien genannt:
Acetyloxy- und Propanoyloxy.

### Cₙ-Alkyloxycarbonyl

Cₙ-Alkyloxycarbonyl steht für die Gruppe -C(O)-O-Cₙ-Alkyl
In der Regel ist n gleich 1 bis 6, bevorzugt 1 bis 5, und besonders bevorzugt 1 bis 4.

### Cₙ-Aromat

Cₙ-Aromat ist ein aromatisches Ringsystem ohne Heteroatom mit n Kohlenstoffatomen.
C₆-Aromat ist gleich Benzol, C₁₀-Aryl ist gleich Naphthalen.

### Cₙ-Aryl

Cₙ-Aryl ist ein monovalentes, aromatisches Ringsystem ohne Heteroatom mit n Kohlenstoffatomen.
C₆-Aryl ist gleich Phenyl. C₁₀-Aryl ist gleich Naphthyl.

### Cₙ-Aryloxy

Cₙ-Aryloxy ist ein Cₙ-Arylether der Formel -O-Cₙ-Aryl.
Bevorzugt ist Phenyloxy.

### Heteroatome

Unter Heteroatomen sind Sauerstoff-, Stickstoff- oder Schwefel-Atome zu verstehen.

### Heteroaromat

Heteroaromat ist ein aromatisches Ringsystem mit mindestens einem von einem Kohlenstoff verschiedenen Heteroatom. Als Heteroatome können Stickstoffatome, Sauerstoffatome und/oder Schwefelatome vorkommen.

Ein monocyclischer Heteroaromat gemäß der vorliegenden Erfindung hat 5 oder 6 Ringatome.

### Heteroaromaten mit 5 Ringatomen umfassen beispielsweise die Ringe:

Thiophen, Thiazol, Furan, Pyrrol, Oxazol, Imidazol, Pyrazol, Isoxazol, Isothiazol, Oxadiazol, Triazol, Tetrazol und Thiadiazol.

### Heteroaromaten mit 6 Ringatomen umfassen beispielsweise die Ringe:

Pyridin, Pyridazin, Pyrimidin, Pyrazin und Triazin.

### Heterozyklus

Heterozyklus im Sinne der Erfindung ist ein vollständig hydrierter Heteroaromat (vollständig hydriertes Heteroaromat = gesättigter Heterozyklus) d.h. ein nicht aromatisches Ringsystem mit mindestens einem von einem Kohlenstoff verschiedenen Heteroatom. Als Heteroatome können Stickstoffatome, Sauerstoffatome und/oder Schwefelatome vorkommen.

### Heterozyklen mit 5 Ringatomen umfassen beispielsweise die Ringe:

Pyrrolidin, Imidazolidin, Pyrazolidin und Tetrahydrofuran.
Heterozyklen mit 6 Ringatomen umfassen beispielsweise die Ringe:
Piperidin, Piperazin, Morpholin, Tetrahydropyran und Thiomorpholin

### Ungesättigter Heterozyklus

Heterozyklus mit mindestens einer Doppelbindung, wobei das Ringsystem nicht aromatisch ist.

### Heterocyclyl

Heterozyklus mit einer freien Bindungsvalenz.

### Halogen

Die Bezeichnung Halogen umfasst Fluor, Chlor, Brom und Iod.
Bevorzugt ist Brom.

### Schutzgruppe

Eine dem Fachmann, insbesondere durch Protective Groups in Organic Chemistry, Third Edition, Theodora W.Greene and Peter G.M. Wuts bekannte Gruppe, die eine funktionelle Gruppe in nachfolgenden Reaktionsschritten schützt.
Beispielsweise sind Cₙ-Alkyl-, Cₙ-Alkenyl oder Cₙ-Alkinylrest, Cₙ-Alkylcarbonyl-, Cₙ-Alkyloxycarbonyl- oder Trialkylsilylreste oder Heterocyclylringe in der Lage, eine Sauerstofffunktion zu schützen.

### Trialkylsilyl

Ein Trialkylsilylrest steht für die Gruppe -SiR¹R²R³, wobei R¹, R², R³ für 3 gleiche oder auch unterschiedliche Cₙ-Alkylreste steht. In der Regel ist n gleich 1 bis 6, bevorzugt 1 bis 4, besonders bevorzugt und beispielhaft seien genannt:
Trimethylsilyl- und *tert*-Butyldimethylsilyl-

Die Erfindung betrifft insbesondere ein Verfahren zur Herstellung von Estra-1,3,5(10)-trienen der Formel (la) aus Verbindungen der Formel (IIa) gemäß

### Schema 2

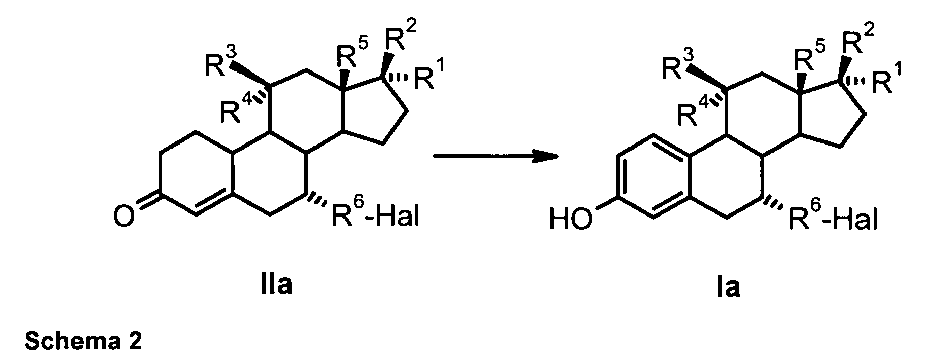

wobei in den Formel (IIa) und (Ia)
- R¹ und R²: zusammen eine Ketogruppe bilden,
oder
- R¹: für Wasserstoff, einen C₁-C₆-Alkyl-, C₂-C₆-Alkenyl- oder C₂-C₆-Alkinylrest steht oder für einen zumindest teilweise fluorierten C₁-C₆-Alkylrest, und
- R²: für Wasserstoff, Hydroxy, C₁-C₆-Alkoxyrest oder einen C₁-C₆-Acyloxyrest steht;
- R³ und R⁴: unabhängig voneinander stehen für Wasserstoff, Halogen und/oder -OR^{B},
wobei R^{B} für Wasserstoff oder eine Schutzgruppe steht,
- R⁵: für einen Methyl- oder Ethylrest steht, und
- R⁶-Hal: für einen C₃-C₁₃-Alkyl-, C₃-C₁₃-Alkenyl- oder C₃-C₁₃-Alkinylrest steht, jeweils mindestes einfach substituiert mit Halogen,
sowie deren Enantiomeren und Diastereomeren.

Besonders geeignet ist das erfindungsgemäße Verfahren zur Herstellung von Verbindungen der allgemeinen Formel (Ib) aus Verbindungen der allgemeinen Formel (IIb) gemäß Schema 3, 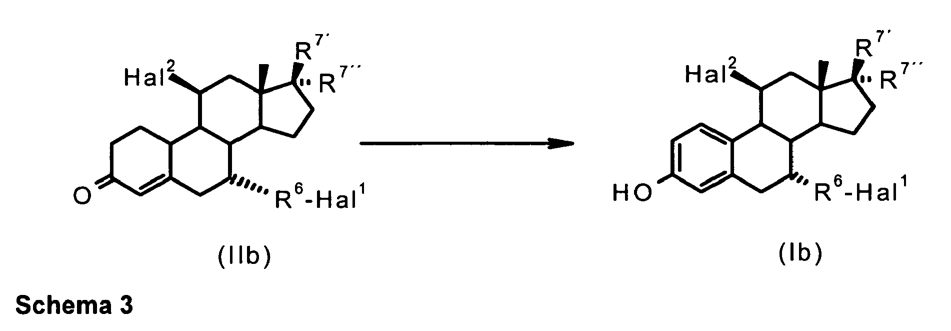 wobei in den Formeln (IIb) und (Ib)
- Hal²: für F oder Cl steht und in 11β-Position an das Estratriengrundgerüst gebunden ist,
- oder:
- R^{7'} und R^{7"}: zusammen eine Ketogruppe bilden,
- R^{7'}: für die Gruppe -OR^{C} steht, wobei R^{C} für Wasserstoff oder eine Schutzgruppe steht,
- R^{7"}: für einen C₁-C₄-Alkylrest oder für einen zumindest teilweise fluorierten C₁-C₄-Alkylrest steht,
wobei R^{7'} in 17β-Position und R^{7"} in 17α-Position an das Estratriengrundgerüst gebunden ist, und
- R⁶-Hal¹: in 7α-Position an das Estratriengerüst gebunden ist und für einen C₃-C₁₁-Alkylrest steht, der mindestens einfach substituiert mit Hal¹, wobei Hal¹ für ein Chlor-, Brom- oder lodatom steht.
sowie deren Enantiomeren und Diastereomeren.

Verbindungen der Formel (IIb) bzw. (Ib), in denen R^{7'} und R^{7"}zusammen eine Ketogruppe bilden, werden im Folgenden als (IIb)-17-Keto-Verbindungen bzw. (Ib)-17-Keto-Verbindungen bezeichnet und in der Formel (IIb-17-Keto) bzw. (Ib-17-Keto) zusammengefasst.

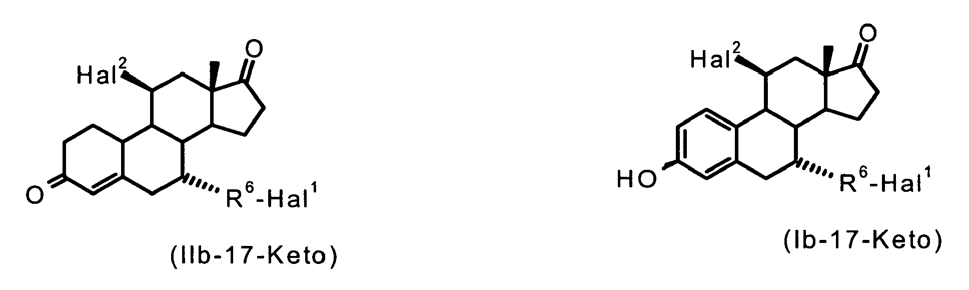

Verbindungen der Formel (IIb) bzw. (Ib), in denen R^{7'} für die Gruppe -OR^{C} steht, wobei R^{C} für Wasserstoff oder eine Schutzgruppe steht, und R^{7"} für einen C₁-C₄-Alkylrest oder für einen zumindest teilweise fluorierten C₁-C₄-Alkylrest steht, werden im Folgenden als (IIb-17β-OR^{C})-Verbindungen bzw. (Ib-17β-OR^{C})-Verbindungen bezeichnet und in der Formel (IIb-17-β- OR^{C}) bzw. (Ib-17β-OR^{C}) zusammengefasst. In IIb-17β-OH-Verbindungen bzw. Ib-17β-OH-Verbindungen liegt die Hydroxygruppe in Position 17 frei vor.

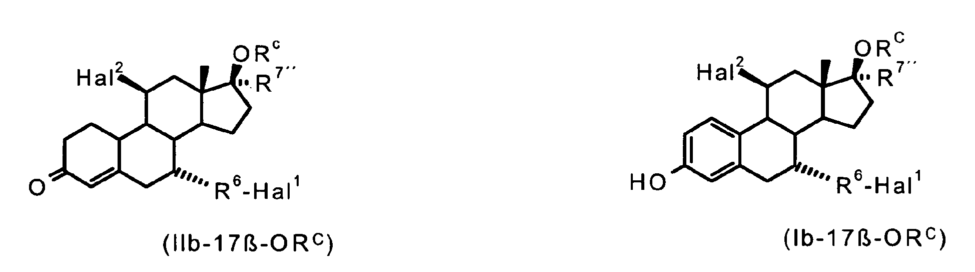

In den Formeln (IIb-17-Keto), (Ib-17-Keto), (IIb-17-β-OR^{C}) bzw. (Ib-17β-OR^{C}) besitzen R⁶-Hal¹ und Hal² die Bedeutungen gemäß Schema 3.

Ganz besonders geeignet ist das erfindungsgemäße Verfahren zur Herstellung von Verbindungen der allgemeinen Formel (Ic) aus Verbindungen der allgemeinen Formel (IIc) gemäß Schema 4, 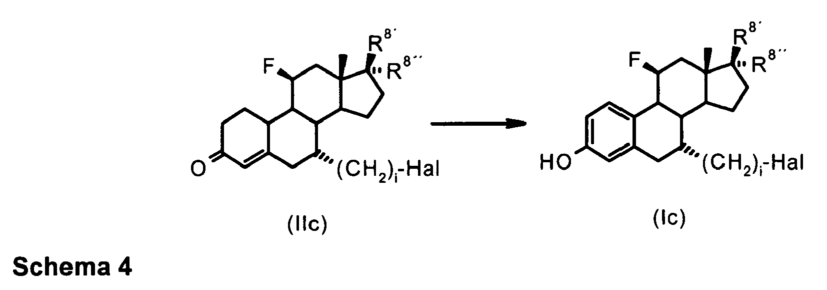 wobei in den Formel (IIc) und (Ic)
- R^{8'} und R^{8"}: zusammen eine Ketogruppe bilden
oder
- R^{8'}: für die Gruppe -OR^{D} steht, wobei R^{D} für Wasserstoff oder eine Schutzgruppe steht
- R^{8"}: für eine Methylgruppe steht,
- i: für eine ganze Zahl von 3 bis 13 steht und
- Hal: für ein Chlor-, Brom- oder Iodatom steht.
sowie deren Enantiomeren und Diastereomeren.

Verbindungen der Formel (IIc) bzw. (Ic), in denen R^{8'} und R^{8"} zusammen eine Ketogruppe bilden, werden im Folgenden als (IIc)-17-Keto-Verbindungen bzw. (Ic)-17-Keto-Verbindungen bezeichnet und in der Formel (IIc-17-Keto) bzw. (Ic-17-Keto) zusammengefasst.

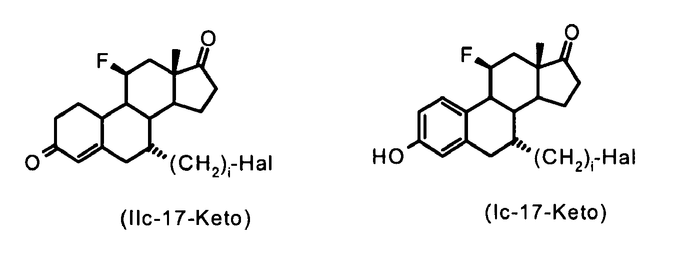

Verbindungen der Formel (IIc) bzw. (Ic), in denen R^{8'} für die Gruppe -OR^{D} steht,
wobei R^{D} für Wasserstoff oder eine Schutzgruppe steht, und R^{8"} für eine Methylgruppe steht, werden im Folgenden als (IIc-17β-OR^{D})-Verbindungen bzw. (Ic-17β-OR^{D})-Verbindungen bezeichnet und in der Formel (IIc-17-β-OR^{D}) bzw. (Ic-17β-OR^{D}) zusammengefasst.
In IIc-17β-OH-Verbindungen bzw. Ic-17β-OH-Verbindungen liegt die Hydroxygruppe in Position 17 frei vor.

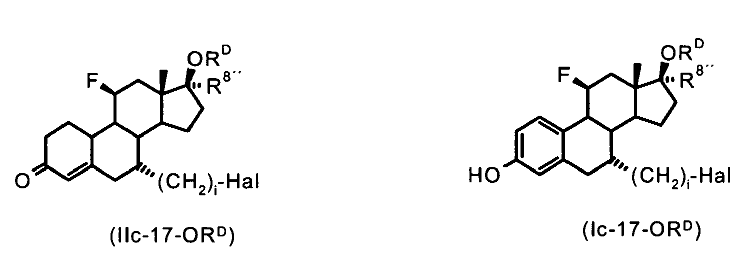

In den Formeln (IIb-17-Keto), (Ib-17-Keto), (IIb-17-β-OR^{D}) bzw. (Ib-17β-OR^{D}) besitzen R^{8"}, Hal, R^{D} und i die Bedeutungen gemäß Schema 4.

Aus Verbindungen der allgemeinen Formel I können Verbindungen mit starker antiestrogener Wirksamkeit hergestellt werden.
So beschreibt WO 03/045972 Estrogenantagonisten, die ihre antiestrogene Wirksamkeit aufgrund der kompetitiven Verdrängung der natürlichen Estrogene von ihrem Rezeptor und/oder durch Destabilisierung des Estrogenrezeptors entfalten. Im letzteren Fall spricht man auch von sogenannten Selektiven Estrogen Rezeptor Destabilisatoren (SERDs). In beiden Fällen wird die Übermittlung des estrogenen Stimulus unterbunden. Auch die Degradation das Estrogenrezeptors kann zur antiestrogenen Wirkung beitragen (Selective Estrogen Receptor Degradation).

Vorzugsweise besitzen die antiestrogenen Verbindungen keine oder nur eine geringe estrogene Restwirkung.

Die erfindungsgemäße Aromatisierung ist unter anderem für die Herstellung von Verbindungen aus WO 03/045972 geeignet.

WO 03/045972 offenbart unter anderem Verbindungen der Formel (Vb). Diese lassen sich gemäß Schema 5 herstellen, 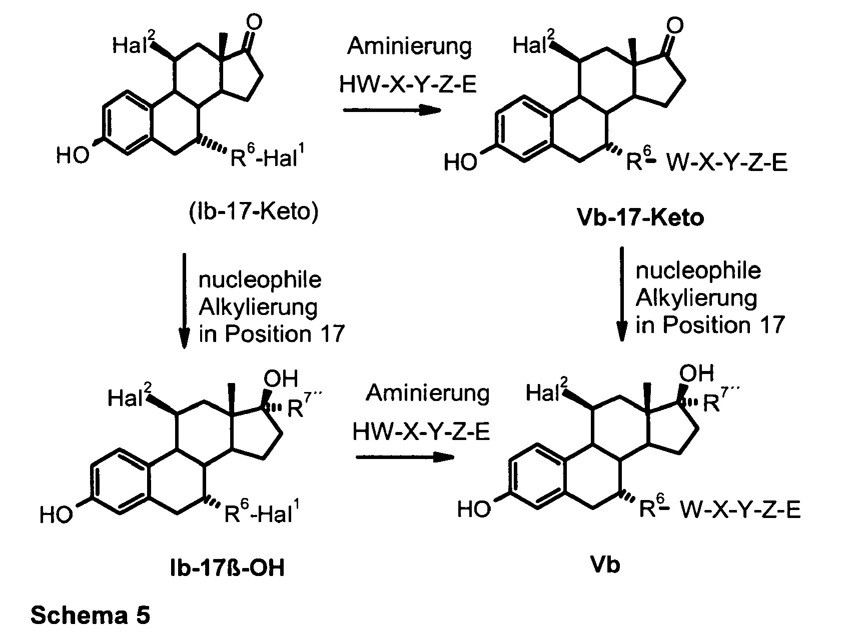 wobei R^{7"}, Hal² und R⁶-Hal¹ die Bedeutungen gemäß Schema 3 besitzen und
- W: für -N(R⁷)- steht, wobei R⁷ Wasserstoff oder ein C₁-C₄-Alkylrest ist,
- X: für -(CH₂)_{q}- steht, wobei q = 0 oder eine ganze Zahl von 1 - 12 ist,
- Y: eine direkte Bindung zwischen X und Z oder eine -SOₙ-Gruppe ist, wobei n = 0,1 oder 2 ist,
- Z: ein gerad- oder verzweigtkettiger C₁-C₇-Alkylenrest ist, der zumindest teilweise fluoriert ist,
- E: für -CF₃ oder für Pentafluorphenyl steht.

Demnach können Verbindungen der Formel (Ib-17-Keto) zunächst mit Aminen der Formel H-W-X-Y-Z-E unter Erhalt von Verbindungen der Formel (Vb-Keto) aminiert werden. Nachfolgend erfolgt die Umsetzung der Verbindungen der Formel (Vb-Keto) in einer nucleophilen Alkylierungsreaktion in Position 17 unter Erhalt von Verbindungen der Formel (Vb).

Alternativ und bevorzugt werden Verbindungen der Formel (Ib-17-Keto) durch eine nucleophile Alkylierungsreaktion in Position 17 zunächst zu Verbindungen der Formel (Ib-17β-OH) umgesetzt. Nachfolgend erfolgt eine Aminierung der Verbindungen der Formel (Ib-17β-OH) mit Aminen der Formel H-W-X-Y-Z-E unter Erhalt von Verbindungen der Formel (Vb).

Die Alkylgruppe in 17α-Position kann jeweils durch übliche Alkylierungsreagenzien, beispielsweise Grignard-Reagentien (CₙAlkyl-Mg-Hal) oder Alkyllithium-Verbindungen (Cₙ-Alkyl-Li) eingeführt werden.

Besonders vorteilhaft ist das erfindungsgemäße Verfahren für die Herstellung der Verbindung AE1: 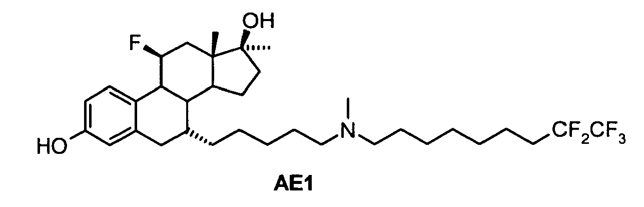 Diese Verbindung wird ebenfalls erstmals in der WO 03/045972 beschrieben.

Die bevorzugten Verbindungen des Typs AE1 (Verbindungen der Formel (Vc)) können gemäß Schema 6 hergestellt werden, 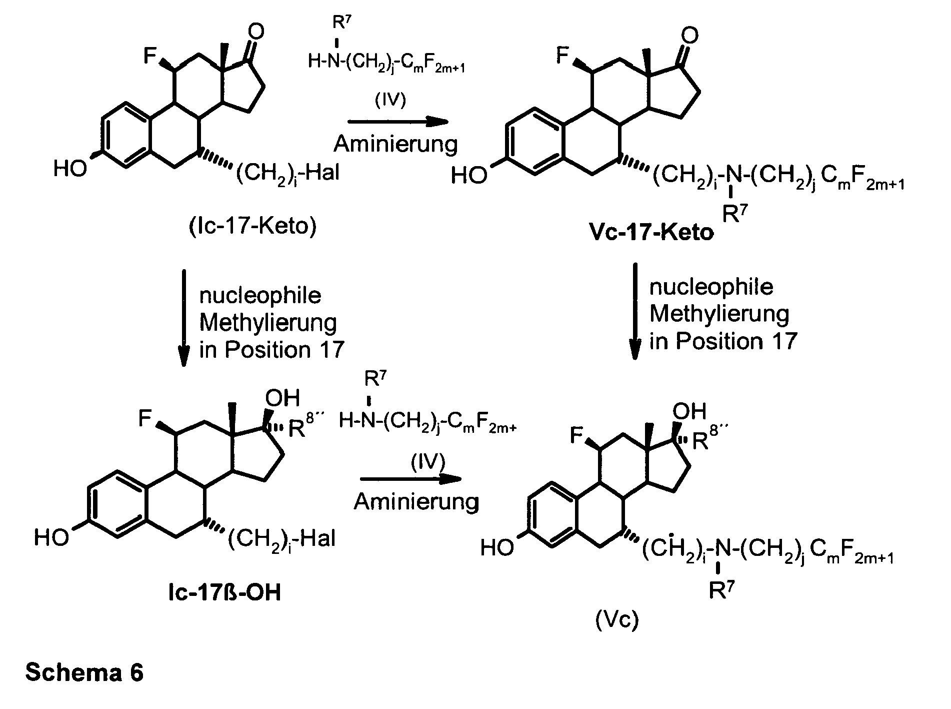 wobei
- i: für eine ganze Zahl von 3 bis 13 steht,
- Hal: für ein Chlor-, Brom- oder lodatom steht,
- R^{8"}: für eine Methylgruppe steht,
- R⁷: für einen C₁-C₄-Alkylrest steht,
- j: für eine ganze Zahl von 1 bis 10 steht und
- m: für eine ganze Zahl von 1 bis 5 steht.

Demnach können Verbindungen der Formel (Ic-17-Keto) zunächst mit α-Alkyl(amin)-ω-perfluor(alkyl)-alkanen der allgemeinen Formel (IV) unter Erhalt von Verbindungen der Formel (Vc-Keto) aminiert werden. Nachfolgend erfolgt die Umsetzung der Verbindungen der Formel (Vc-Keto) in einer nucleophilen Alkylierungsreaktion in Position 17 unter Erhalt von Verbindungen der Formel (Vc).

Alternativ und bevorzugt werden Verbindungen der Formel (Ic-17-Keto) durch eine nucleophile Alkylierungsreaktion in Position 17 zunächst zu Verbindungen der Formel (Ic-17β-OH) umgesetzt. Nachfolgend erfolgt eine Aminierung der Verbindungen der Formel (Ic-17β-OH) mit α-Alkyl(amin)-ω-perfluor(alkyl)-alkanen der allgemeinen Formel (IV) unter Erhalt von Verbindungen der Formel (Vc).

Die Methylgruppe in 17α-Position kann jeweils durch übliche Alkylierungsreagenzien, beispielsweise Grignard-Reagentien (Methyl-MgHal) oder Methyllithium eingeführt werden.

Ein möglichen Weg zur Aromatisierung des A-Ringes ist in WO 99/33855 offenbart:

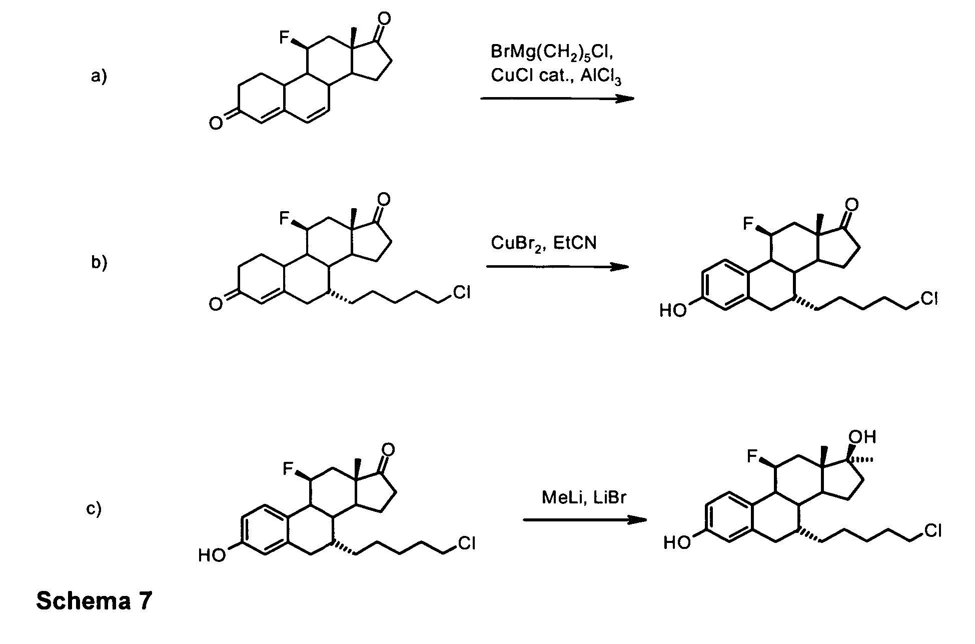

Die Aromatisierung des A-Ringes in Teilschritt b) erfolgt nach diesem Stand der Technik durch eine CuBr₂-vermittelte Oxidation.

Nachteilig an der Durchführung des Teilschrittes b) gemäß WO99/33855 ist, dass unter den bisher bekannten Bedingungen der Reaktionsführung in einer Folgereaktion bromierte Nebenprodukte entstehen, die die Ausbeute an Verbindungen der Formel (I) reduzieren und vom Produkt schwer abtrennbar sind.

### Beispielhaft sei folgende Neben/Folgereaktion genannt:

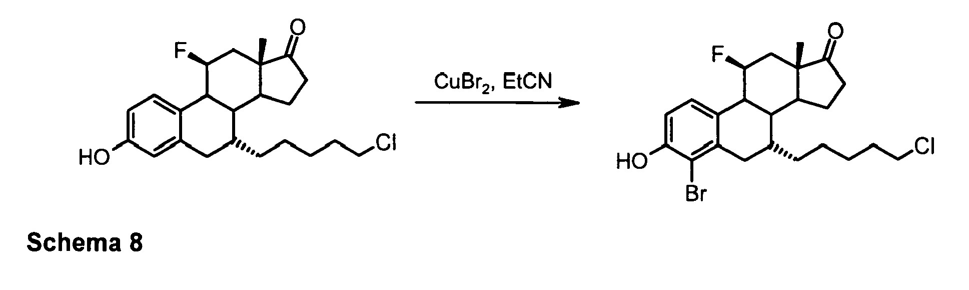

Das Bromierungs-Produkt ist eine unerwünschte Verunreinigung, und diese muss im weiteren Verlauf der Synthese abgetrennt werden - entweder durch aufwändige Chromatographie oder durch wiederholte Kristallisation einer Zwischenstufe bzw. des Wirkstoffs.

Aus der Literatur (WO2007/049672 und Steroids 1994, vol.59, S.621ff*.)* ist bekannt, dass vergleichbare Oxidationen steroidaler Systeme des allgemeinen Typs (II) zu steroidalen Systemen des allgemeinen Typs (I) unter Verwendung von unterschiedlichen Mengen an CuBr₂ durchgeführt werden können.

Die Folge bzw. Nebenreaktion ist auch in WO 02/32922 beschrieben. Zur Minimierung der Bromierung wird dort ein Verfahren unter Zusatz von EssigsäureAnhydrid vorgeschlagen.
Das Verfahren gemäß WO 02/32922 hat allerdings folgende Nachteile:
- es muss mit mehr als 2 Equivalenten CuBr₂ (2Equivalenten.=stöchiometrisch) gearbeitet werden d.h. eine Luftoxidation wird nicht genutzt, wodurch das Verfahren kostenintensiv wird,
- es entsteht nicht das gewünschte "freie Phenol", sondern das 3-Acetat. Dieses 3-Acetat muss anschließend in einem weiteren Reaktionsschritt zum gewünschten Produkt verseift werden,
- die Abtrennung der großen Mengen an Cu-Salzen macht einen weiteren Verfahrensschritt erforderlich.

Ausgehend von diesem Stand der Technik besteht die Aufgabe der vorliegenden Erfindung darin, ein Verfahren zur Herstellung von Verbindungen der allgemeinen Formel (I) bereitzustellen, das die Bildung von bromierten Neben- bzw. Folgeprodukten unterdrückt, um vor allem die Ausbeute zu erhöhen, um zusätzliche Aufreinigungsschritte zu vermeiden und anfallende Mengen an Kupfer-haltigen Abwässern zu verringern.

Vor allem sollen bromierte Nebenprodukte des Formel (I-n) vermieden werden. 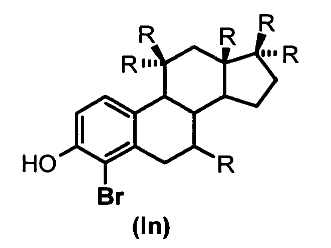 wobei alle R unabhängig voneinander für chemisch stabile beliebige Reste stehen.

So sollen bei der Herstellung von Verbindungen der Formel (la) möglichst wenige Nebenprodukte der Formel (Ia-n), bei Verbindungen der Formel (Ib) möglichst wenige Nebenprodukte der Formel (Ib-n) und bei Verbindungen der Formel (Ic) möglichst wenige Nebenprodukte der Formel (Ic-n) entstehen. 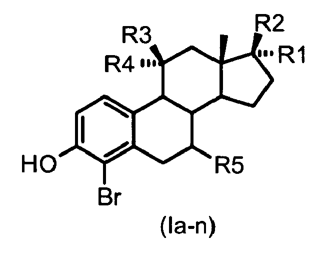 mit R¹, R², R³, R⁴ und R⁵ definiert wie in Formel (la) 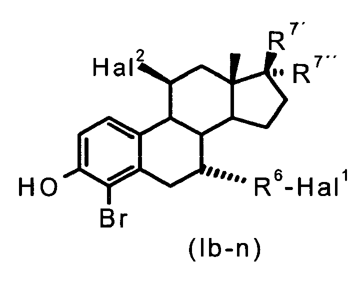 mit R^{7'}, R^{7"}, R⁶-Hal¹ und Hal² definiert wie in Formel (Ib) 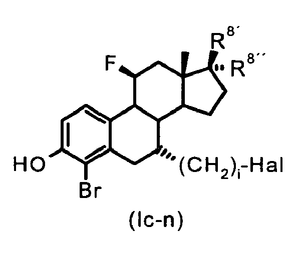 mit i, R^{8'}, R^{8"} und Hal definiert wie in Formel (Ic) Darüber hinaus soll das CuBr₂ in maximal stöchiometrischen Mengen eingesetzt werden müssen.

Bevorzugt soll das Verfahren durch den Zusatz von Säuren so beschleunigt werden, dass nahezu vollständige bis vollständige Umsätze auch mit substöchiometrischen Mengen CuBr₂ erreicht werden.

Die Oxidation soll ebenso bevorzugt in Kombination mit einer Luftoxidation durchgeführt werden.

Die Aufgabe der vorliegenden Erfindung wird dadurch gelöst, dass die CuBr₂ vermittelte Aromatisierung des Ringes A in Gegenwart von mindestens einem elektronenreichen, ungesättigten organischen Zusatz erfolgt.

Als elektronenreiche, ungesättigte organische Zusätze sind Substanzen der allgemeinen Formel (Z) geeignet 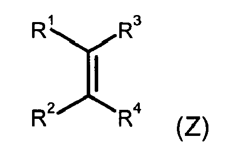 wobei
- R¹, R², R³, R⁴: unabhängig voneinander für Wasserstoff, einen C₁-C₆-Alkyl-, C₂-C₆-Alkenyl-, C₁-C₆-Alkoxy- oder C₂-C₆-Alkenoxyrest oder einen C₆-Arylring oder C₆-Aryloxyring stehen, mit der Maßgabe, dass mindestens ein Rest von R¹, R², R³, R⁴ ungleich Wasserstoff ist,
- oder:
- R¹ und R²: bilden zusammen mit den C-Atomen der Doppelbindung einen gegebenenfalls ein- oder mehrfach, gleich oder verschieden mit Hydroxy, einem C₁-C₆-Alkoxy- und/oder C₁-C₆-Alkylrest substituierten C₆-Aromaten, ein C₃-C₇-Cycloalken, einen monocyclischen Heteroaromaten oder einen ungesättigten Heterozyklus, der gegebenenfalls zusätzlich mit R³ und R⁴ substituiert ist, wobei R³ und R⁴ unabhängig voneinander stehen für einen C₁-C₆-Alkyl-, C₂-C₆-Alkenyl-, C₁-C₆-Alkoxy- oder C₂-C₆-Alkenoxyrest oder einen C₆-Arylring oder C₆-Aryloxyring.

Bevorzugt werden von R¹ und R² mehrfach mit C₁-C₆-Alkoxyresten substituierte C₆-Aromaten gebildet.
Zu dieser bevorzugten Untergruppe an Zusätzen gehören als besonders bevorzugt 1,3,5-Trimethoxybenzol und 1,3-Dimethoxybenzol.

Bevorzugt werden von R¹ und R² auch ungesättigte Cycloalkene gebildet.
Zu dieser bevorzugten Untergruppe an Zusätzen gehört als besonders bevorzugt Cyclohexen.

Bevorzugt werden von R¹ und R² auch sauerstoffhaltige, ungesättigte Heterozyklen gebildet.
Zu dieser bevorzugten Untergruppe an Zusätzen gehört als besonders bevorzugt Dihydrofuran.

Vom elektronenreichen ungesättigten Zusatz werden 0.1 bis 3 Equivalente in Bezug auf das eingesetzte Steroid eingesetzt, bevorzugt 0.5 bis 2.0 Equivalente und besonders bevorzugt 1 Equivalent.

Im allgemeinen wird das Steroid zusammen mit einer geeigneten Menge an CuBr₂ und LiBr umgesetzt. Dies geschieht in einem polar-aprotischen Lösungsmittel, vorzugsweise Acetonitril oder Propionitril.
1 Equivalent des Steroids wird mit 0.1 bis 2.0 Equivalenten CuBr₂ und 0.1 bis 5.0 Equivalenten LiBr umgesetzt. Bevorzugt werden 0.5 bis 1.3 Equivalente CuBr₂ und 0.5 bis 3.0 Equivalenten LiBr eingesetzt. Besonders bevorzugt sind 0.5 Equivalente CuBr₂ und 1.0 Equivalent LiBr.

Zusätzlich wird eine geeignete Menge einer Säure der allgemeinen Formel H⁺R⁻ zugegeben - wobei R⁻ = Hal⁻, -SO₂-Alkyl, -SO₂-Aryl sein kann.
Bevorzugt ist Methansulfonsäure.

Geeignet sind auch Zusätze, die Säuren freisetzen wie z.B. Trimethylsilylbromid oder Trimethylsilylchlorid, aus denen HBr bzw. HCl freigesetzt wird.

Die Säure wird in einer Menge von 0.1 bis 2.0 Equivalenten, bevorzugt 0.2-0.6 Equivalenten zugesetzt.

Zur vollständigen Oxidation des Edukts wird anschließend Luft - vorzugsweise ein Gemisch aus Stickstoff und Sauerstoff - bis zur vollständigen Umsetzung durch das Reaktionsgemisch geleitet.

Das Verfahren kann bei unterschiedlichen Temperaturen gefahren werden, vorzugsweise im Bereich zwischen +10°C bis +50°C.

Durch einen elektronenreichen ungesättigten organischen Zusatz wird die Bromierung des Steroids verhindert.

In der Reaktionsmischung des Beispiels 1 lässt sich beispielsweise durch HPLC-Analytik die Bildung von 4-Brom-1-3-dimethoxybenzol und die Abnahme von 1,3-Dimethoxybenzol verfolgen. Damit wird klar, dass bedingt durch die unterschiedlichen Reaktivitäten der beiden elektronenreichen Aromaten (steroidales Phenol vs 1-3-Dimethoxybenzol) bzgl. einer Bromierung zunächst das reaktivere 1,3-Dimethoxybenzol bromiert wird (siehe Schema 9) und die ungewünschte Bromierung des steroidalen aromatischen Systems zurückgedrängt wird. Das gewünschte steroidale Produkt ist stabil in der Reaktionsmischung. 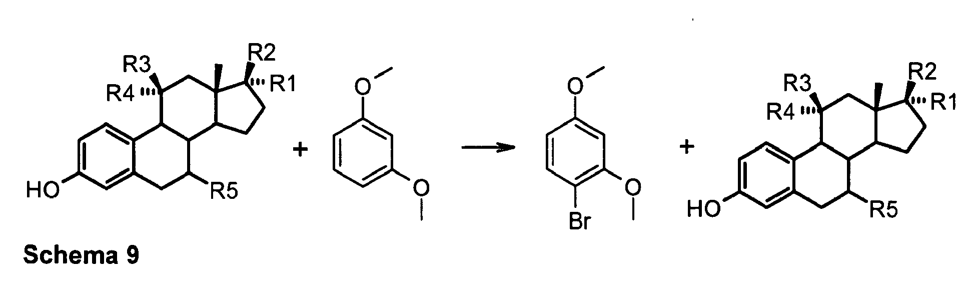 In der Literatur sind bisher keine Beispiele zu finden, in denen eine Aromatisierung steroidaler Systeme des allgemeinen Typs (II) unter Verwendung von CuBr₂ und in Gegenwart von elektronenreichen ungesättigten Zusätzen durchgeführt wird. Hierbei kann die Umsetzung durch den Zusatz einer Säure beschleunigt werden und mit einer Luftoxidation kombiniert werden.

### Beispiele

### Beispiel 1:

Synthese von **7-α-(5-Chlorpentyl)-11-β-fluor-3-hydroxyestra-1,3,5(10)-trien-17-on (I-1) aus 7-α-(5-Chlorpentyl)-11-β-fluor-3-hydroxyestr-4-en-3,17-dion (II-1)** 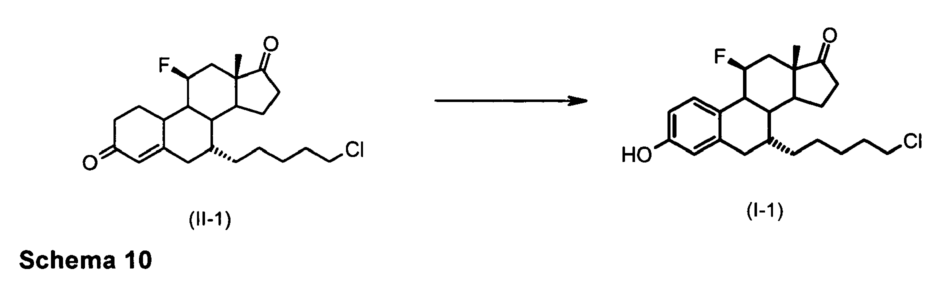 10.37 g (26.26 mmol) 7-α-(5-Chlorpentyl)-11-β-fluor-3-hydroxyestr-4-en-3,17-dion werden in 52 ml Propionitril zusammen mit 3.42 ml (26.26 mmol) 1,3-Dimethoxybenzol vorgelegt. Bei RT wird dazu eine Lösung aus 2.93 g (13.13 mmol) CuBr₂ und 2.28 g (26,26 mmol) LiBr in 52 ml Proprionitril gegeben. Durch das Reaktionsgemisch wird ein Gemisch bestehend aus 70% (v/v) Stickstoff und 30% (v/v) Sauerstoff geleitet. Das Reaktionsgemisch wird auf 40°C erwärmt und in 2 h mit einer Lösung aus 0.85 ml (13.13 mmol) Methansulfonsäure in 45 ml Proprionitril versetzt. Es wird bis zum vollständigen Umsatz gerührt (ca. 4 h).
Anschließend wird das Reaktionsgemisch durch Zugabe von 40 ml 20%iger wäßriger K2HPO4-Lösung gequenscht. Während des Nachrührens wird der pH-Wert kontrolliert und ggf. auf pH = 7 angepasst. Die Suspension wird über einen mit Celite bedeckten Druckfilter filtriert und dieser wird mit ca. 120 ml Toluol substanzfrei gewaschen. Nach Abtrennung der Wasserphase wird die org. Phase 2 x mit einer Lösung bestehend aus 10 g Natriumedetat und 1 g Natriumhydroxid in 100 ml Wasser zur Entfernung des Kupfers extrahiert. Die Produktlösung wird vollständig eingeengt. Es werden 15.51 g Rohprodukt isoliert. Zusammensetzung des Rohprodukts laut HPLC (Prontosil C18-ace-EPS; 1mL/min Wasser/ACN + 0.1% HCOOH; 215 nm): 82.5% 1-1, 0.0% I-2, 17.5% Sonstige.

### Screening von Reaktionsbedingungen und Scavengern in kleinem Reaktionsmaßstab an II-1

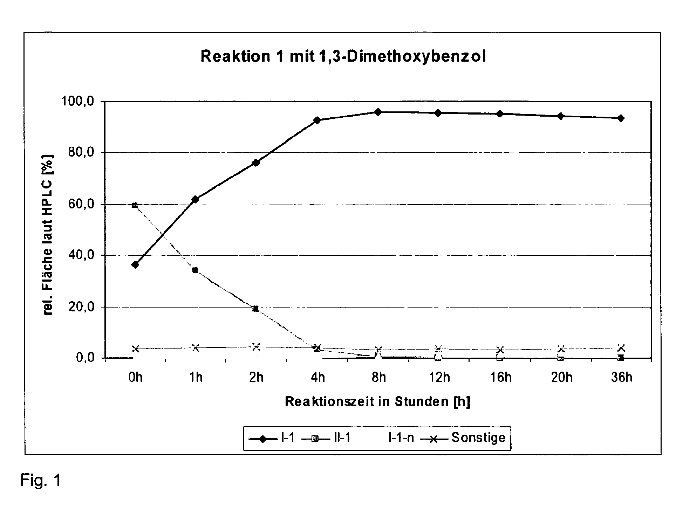

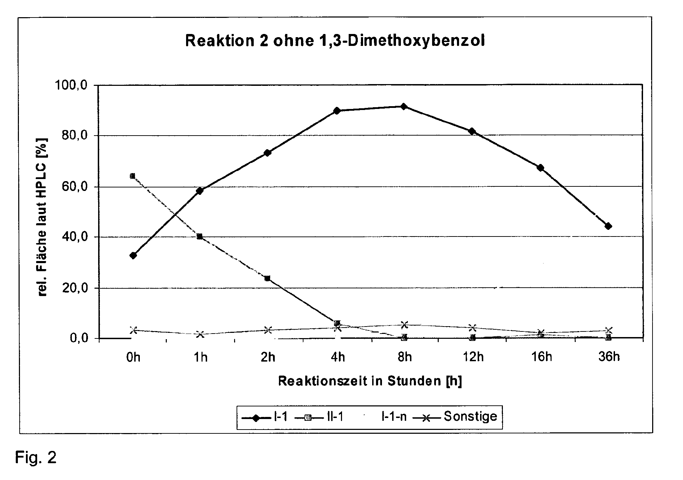

Bei der Entwicklung des beschriebenen Verfahrens wurden zunächst eine Vielzahl von Reaktionsbedingungen und möglichen Scavengern in einem kleinen Maßstab von nur 100mg 7-α-(5-Chlorpentyl)-11-β-fluor-3-hydroxyestr-4-en-3,17-dion untersucht. In diesem Reaktionsmaßstab reicht bereits das einfache Rühren im offenen Schraubdeckelgläschen im Kontakt mit der Raumluft, um eine Oxidation zu bewirken ohne dass speziell Sauerstoff eingeleitet werden muß. Dies führte in nachfolgend beschriebenen Screeningexperimenten zu längeren Reaktionszeiten als im ausgearbeiteten Verfahren, was aber eine bessere Beobachtung des Reaktionsverlaufs erlaubt. Die Unterdrückung von Nebenverbindungen durch Zusatz von 1,3-Dimethoxybenzol kann bereits durch diese Screeningexperimente im kleinen Maßstab deutlich gezeigt werden. Exemplarisch werden deshalb Ergebnisse aus zwei Experimenten wiedergegeben. Nach den oben aufgeführten Bedingungen wurde - durch fortlaufende Entnahme von HPLC-Proben (100%-Methode) - ein Vergleich der Reaktionsverläufe mit (Reaktion 1: Tab. 1, Fig 1), bzw. ohne Zugabe von 1,3-Dimethoxybenzol (Reaktion 2: Tab.2, Fig.2) durchgeführt.
Für Reaktion 1 und Reaktion 2 wurden 100 mg (0.25 mmol) 7-α-(5-Chlorpentyl)-11-β-fluor-3-hydroxyestr-4-en-3,17-dion, 28mg (0.13 mmol) CuBr₂, 22 mg (0.25 mmol) LiBr in 1.5mL Propionitril vorgelegt, mit 16 µL (0.25 mmol) Methansulfonsäure versetzt und im offenen Gefäß gerührt. Reaktion 1 wurde zusätzlich mit 99 µL (0.75 mmol) 1,3-Dimethoxybenzol versetzt. Es wurden zu den angegebenen Zeiten HPLC-Proben entnommen. Die analytischen Ergebnisse sind in den folgenden Tabellen wiedergegeben:

**Tab.1: HPLC- Daten zu Reaktion 1**

| **Reaktionszeit in Stunden [h]** | **I-1 [%]** | **II-1 [%]** | **I-1-n [%]** | **Sonstige [%]** |
|---|---|---|---|---|
| 0h | 36,5 | 59,7 | 0,0 | 3,8 |
| 1h | 62,0 | 34,1 | 0,0 | 3,9 |
| 2h | 76,3 | 19,1 | 0,0 | 4,6 |
| 4h | 92,7 | 3,2 | 0,0 | 4,1 |
| 8h | 95,8 | 0,2 | 0,6 | 3,4 |
| 12h | 95,5 | 0,0 | 1,0 | 3,5 |
| 16h | 95,2 | 0,0 | 1,6 | 3,2 |
| 20h | 94,4 | 0,0 | 1,8 | 3,8 |
| 36h | 93,3 | 0,0 | 2,8 | 3,8 |

**Tab.2: HPLC-Daten zu Reaktion 2**

| **Reaktionszeit in Stunden [h]** | **I-1 [%]** | **II-1 [%]** | **I-1-n [%]** | **Sonstige [%]** |
|---|---|---|---|---|
| 0h | 32,9 | 64,0 | 0,0 | 3,2 |
| 1 h | 58,3 | 40,0 | 0,0 | 1,7 |
| 2h | 73,4 | 23,4 | 0,0 | 3,2 |
| 4h | 90,0 | 5,7 | 0,2 | 4,1 |
| 8h | 91,5 | 0,0 | 3,4 | 5,1 |
| 12h | 82,0 | 0,0 | 14,1 | 3,9 |
| 16h | 67,4 | 1,2 | 29,2 | 2,2 |
| 36h | 44,2 | 0,0 | 52,9 | 2,9 |

### Beispiel 2:

### Synthese von 8-α-Estron (I-2) aus 4-Estren-3,17-dion ((II-2))

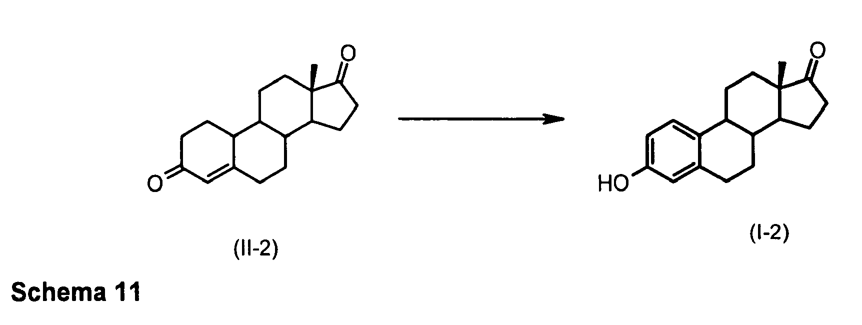

Eine Lösung von 69mg (0.25mmol) 4-Estren-3,17-dion (II-2) in 1.5mL Propionitril wird bei Raumtemperatur zu 33mg (0.14mmol) CuBr₂ und 28mg (0.32mmol) LiBr in offenen 8mL Vials gegeben und 2min geschüttelt. Dann werden 0.5mL einer Lösung von 4.9mg (0.05 mmol) Methansulfonsäure in Propioniril und 0.2mL einer Lösung von 70mg (0.51 mmol) 1,3-Dimethoxybenzol in Propionitril zugegeben. Das Reaktionsgefäss wird offen - d.h. unter Kontakt der Reaktionsmischung mit der Raumluft - geschüttelt und Proben für die HPLC entnommen. Zusammensetzung der Reaktionsmischung nach 8h laut HPLC (Phenomenex Synergi Polar-RP 4µ; 1mL/min Wasser/ACN + 0.1 % HCOOH; 220nm): 91.7% I-2, 1.8% II-2, 6.5% Sonstige.

### Beispiel 3:

### Synthese von 11-α-Acetyloxy-3-hydroxyestra-1,3,5(10)-trien-17-on (I-3) aus 11-α-Acetyloxyestr-4-en-3,17-dion (II-3)

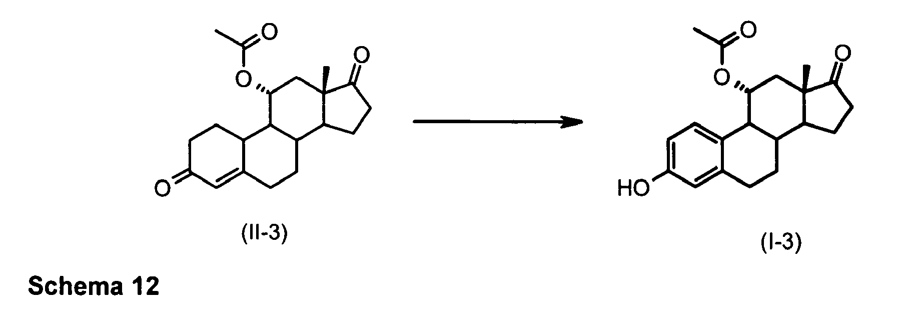

Eine Lösung von 84mg (0.25mmoi) 11-α-Acetyloxyestr-4-en-3,17-dion (II-3) in 1.5mL Propionitril und 1.5mL Dichlormethan wird bei Raumtemperatur zu 33mg (0.14mmol) CuBr₂ und 28mg (0.32mmol) LiBr in offenen 8mL Vials gegeben und 2min geschüttelt. Dann werden 0.5mL einer Lösung von 4.9mg (0.05 mmol) Methansulfonsäure in Propioniril und 0.2mL einer Lösung von 70mg (0.51 mmol) 1,3-Dimethoxybenzol in Propionitril zugegeben. Das Reaktionsgefäss wird offen - d.h. unter Kontakt der Reaktionsmischung mit der Raumluft - geschüttelt und Proben für die HPLC entnommen. Zusammensetzung der Reaktionsmischung nach 6h laut HPLC (Phenomenex Synergi Polar-RP 4µ; 1 mL/min Wasser/ACN + 0.1 % HCOOH; 220nm): 93.1% I-3, 0.0% II-3, 6.9% Sonstige.

### Beispiel 4:

### Synthese von 11-β-Fluor-3-hydroxyestra-1,3,5(10)-trien-17-on (I-4) aus 11-β-Fluorestr-4-en-3,17-dion (II-4)

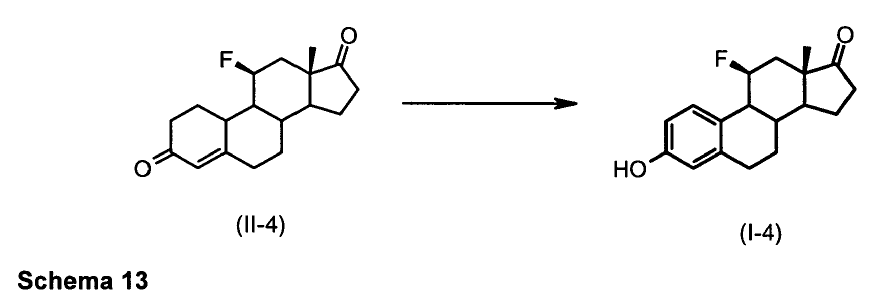

Eine Lösung von 74mg (0.25mmol) 11-β-Fluorestr-4-en-3,17-dion (II-4) in 1.5mL Propionitril wird bei Raumtemperatur zu 33mg (0.14mmol) CuBr₂ und 28mg (0.32mmol) LiBr in offenen 8mL Vials gegeben und 2min geschüttelt. Dann werden 0.5mL einer Lösung von 4.9mg (0.05mmol) Methansulfonsäure in Propioniril und 0.2mL einer Lösung von 70mg (0.51 mmol) 1,3-Dimethoxybenzol in Propionitril zugegeben. Das Reaktionsgefäss wird offen - d.h. unter Kontakt der Reaktionsmischung mit der Raumluft - geschüttelt und Proben für die HPLC entnommen. Zusammensetzung der Reaktionsmischung nach 18h laut HPLC (Phenomenex Synergi Polar-RP 4µ; 1 mL/min Wasser/ACN + 0.1 % HCOOH; 220nm): 78.0 I-4, 6.81-4, 15.2% Sonstige.

### Beispiel 5:

### Synthese von 17-β-Acetoxy-1,3,5(10)-estratrien-3-ol (II-5) aus 17-b-Acetoxy-4-estren-3-on (I-5)

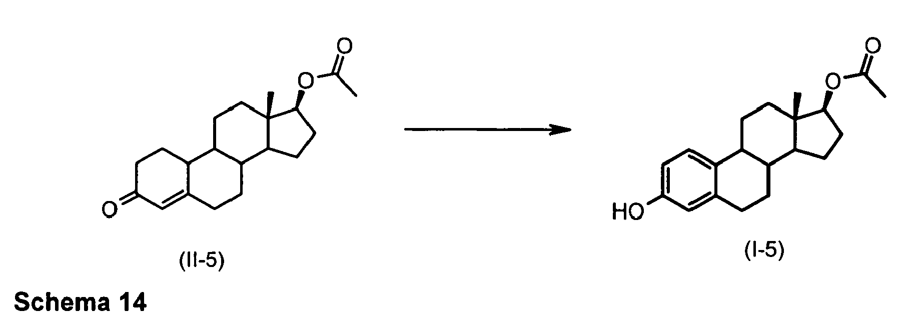

Eine Lösung von 80mg (0.25mmol) 17-b-Acetoxy-4-estren-3-on (II-5) in 1.5mL Propionitril wird bei Raumtemperatur zu 33mg (0.14mmol) CuBr₂ und 28mg (0.32mmol) LiBr in offenen 8mL Vials gegeben und 2min geschüttelt. Dann werden 0.5mL einer Lösung von 4.9mg (0.05 mmol) Methansulfonsäure in Propioniril und 0.2mL einer Lösung von 70mg (0.51 mmol) 1,3-Dimethoxybenzol in Propionitril zugegeben. Das Reaktionsgefäss wird offen - d.h. unter Kontakt der Reaktionsmischung mit der Raumluft - geschüttelt und Proben für die HPLC entnommen. Zusammensetzung der Reaktionsmischung nach 18h laut HPLC (Phenomenex Synergi Polar-RP 4µ; 1 mL/min Wasser/ACN + 0.1 % HCOOH; 220nm): 85.4% I-5, 2.1% II-5, 87.5% Sonstige.

### Beschreibung der Figuren

### Fig. 1

Graphische Darstellung der HPLC-Daten von Reaktion 1 aus Tab. 1. Im Beobachtungszeitraum von 36h ist keine wesentliche Zersetzung des Reaktionsprodukts I-1 zum bromiertenNebenprodukt I-1-n zu beobachten.

| | | |
|---|---|---|
| -◆- | = | I-1; |
| -■- | = | II-2; |
| -▲- | = | I-1-n, |
| -x- | = | Sonstige |

### Fig.2

Graphische Darstellung der HPLC-Daten von Reaktion 2 aus Tab. 2. Im Beobachtungszeitraum von 36h ist eine deutliche Zersetzung des Reaktionsprodukts I-1 zum bromiertenNebenprodukt I-1-n zu beobachten.

| | | |
|---|---|---|
| -◆- | = | I-1; |
| -■- | = | II-2; |
| -▲- | = | I-1-n, |
| -x- | = | Sonstige |

## Patentansprüche

1. Verfahren zur CuBr₂ vermittelten Aromatisierung von 19-Nor-androst-4-en-3-onen der allgemeinen Formel (II) zu Estra-1,3,5(10)-trienen der allgemeinen Formel I 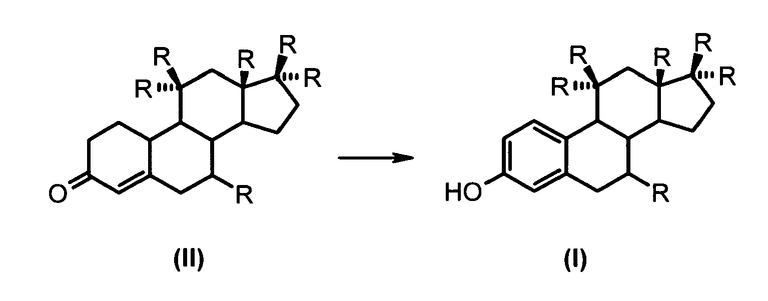 wobei in Formel (II) und Formel (I) alle R unabhängig voneinander für chemisch stabile beliebige Reste stehen, **dadurch gekennzeichnet dass** die Aromatisierung in Gegenwart von mindestens einem elektronenreichen, ungesättigten organischen Zusatz der allgemeinen Formel (Z) erfolgt 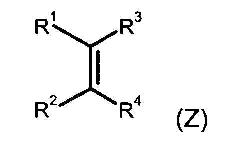 wobei
R¹, R², R³, R⁴ unabhängig voneinander für Wasserstoff, einen C₁-C₆-Alkyl-, C₂-C₆-Alkenyl-, C₁-C₆-Alkoxy- oder C₂-C₆-Alkenoxyrest oder einen C₆-Arylring oder C₆-Aryloxyring stehen, mit der Maßgabe, dass mindestens ein Rest von R¹, R², R³, R⁴ ungleich Wasserstoff ist,
oder
R¹ und R² bilden zusammen mit den C-Atomen der Doppelbindung einen gegebenenfalls ein- oder mehrfach, gleich oder verschieden mit Hydroxy, einem C₁-C₆-Alkoxy- und/oder C₁-C₆-Alkylrest substituierten C₆-Aromaten, ein C₃-C₇-Cycloalken, einen monocyclischen Heteroaromaten oder einen ungesättigten Heterozyklus, der gegebenenfalls zusätzlich mit R³ und R⁴ substituiert ist, wobei R³ und R⁴ unabhängig voneinander stehen für einen C₁-C₆-Alkyl-, C₂-C₆-Alkenyl-, C₁-C₆-Alkoxy- oder C₂-C₆-Alkenoxyrest oder einen C₆-Arylring oder C₆-Aryloxyring.
sowie deren Enantiomeren und Diastereomeren.

2. Verfahren gemäß Anspruch 1 zur Herstellung von Estra-1,3,5(10)-trienen der Formel (la) aus Verbindungen der Formel (IIa), 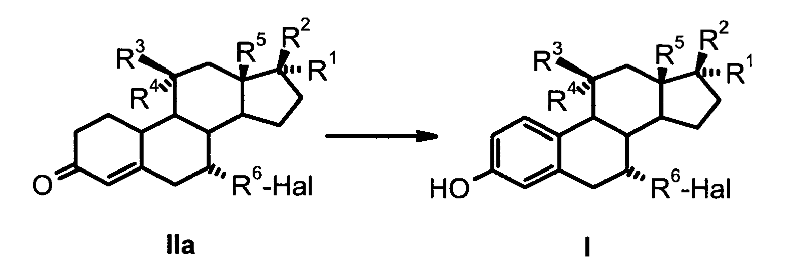 wobei
R¹ und R² zusammen eine Ketogruppe bilden,
oder
R¹ für Wasserstoff, einen C₁-C₆-Alkyl-, C₂-C₆-Alkenyl- oder C₂-C₆-Alkinylrest steht oder für einen zumindest teilweise fluorierten C₁-C₆-Alkylrest, und
R² für Wasserstoff, Hydroxy, C₁-C₆-Alkoxyrest oder einen C₁-C₆-Acyloxyrest steht;
R³ und R⁴ unabhängig stehen für Wasserstoff, Halogen und/oder -OR^{B}, wobei R^{B} für Wasserstoff oder eine Schutzgruppe steht,
R⁵ für einen Methyl- oder Ethylrest steht, und
R⁶-Hal für einen C₃-C₁₃-Alkyl-, C₃-C₁₃-Alkenyl- oder C₃-C₁₃-Alkinylrest steht, jeweils mindestes einfach substituiert mit Halogen,
sowie deren Enantiomeren und Diastereomeren.

3. Verfahren gemäß Anspruch 1 zur Herstellung von Verbindungen der allgemeinen Formel (Ib) aus Verbindungen der allgemeinen Formel (IIb), 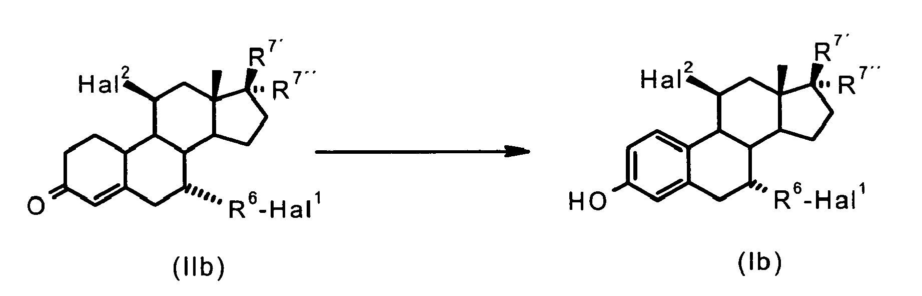 wobei
Hal² für F oder Cl steht und in 11 β-Position an das Estratriengrundgerüst gebunden ist,
R^{7'} und R^{7"} zusammen eine Ketogruppe bilden,
oder
R^{7'} für die Gruppe -OR^{C} steht, wobei R^{C} für Wasserstoff oder eine Schutzgruppe steht,
R^{7"} für einen C₁-C₄-Alkylrest oder für einen zumindest teilweise fluorierten C₁-C₄-Alkylrest steht,
wobei R^{7'} in 17β-Position und R^{7"} in 17α-Position an das Estratriengrundgerüst gebunden ist, und
R⁶-Hal¹ in 7α-Position an das Estratriengerüst gebunden ist und für einen C₃-C₁₁-Alkylrest steht, der mindestens einfach substituiert mit Hal¹, wobei Hal¹ für ein Chlor-, Brom- oder lodatom steht.
sowie deren Enantiomeren und Diastereomeren.

4. Verfahren gemäß Anspruch 1 zur Herstellung von Verbindungen der allgemeinen Formel (Ic) aus Verbindungen der allgemeinen Formel (IIc), 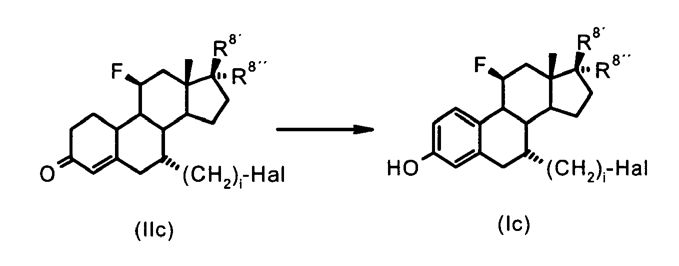 in dem
R^{8'} und R^{8"} zusammen eine Ketogruppe bilden
oder
R^{8'} für die Gruppe -OR^{D} steht, wobei R^{D} für Wasserstoff oder eine
Schutzgruppe steht
R^{8"} für eine Methylgruppe steht,
i für eine ganze Zahl von 3 bis 13 steht und
Hal für ein Chlor-, Brom- oder lodatom steht.
sowie deren Enantiomeren und Diastereomeren.

5. Verfahren nach einem der Ansprüche 1 bis 4,
wobei als Zusatz 1,3,5-Trimethoxybenzol und/oder 1,3-Dimethoxybenzol eingesetzt wird.

6. Verfahren nach einem der Ansprüche 1 bis 4,
wobei als Zusatz Cyclohexen eingesetzt wird.

7. Verfahren nach einem der Ansprüche 1 bis 4,
wobei als Zusatz Dihydrofuran eingesetzt wird.

8. Verfahren zur Herstellung von Verbindungen der allgemeinen Formel (Vb), mit den Teilschritten
a) Aromatisierung einer Verbindung der allgemeinen Formel (IIb-17-Keto) zu einer Verbindung der allgemeinen Formel (Ib-17-Keto) gemäß Anspruch 3 oder einem der Ansprüche 4 bis 7 in Abhängigkeit von Anspruch 3, 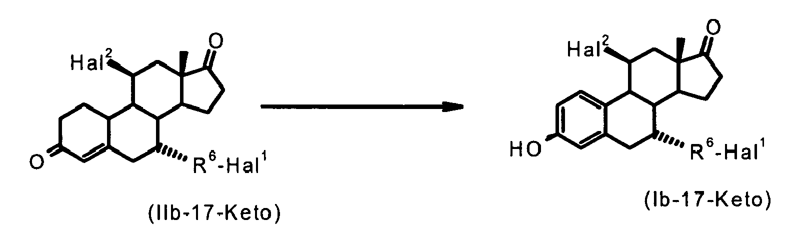
b) Umsetzung der Verbindungen der allgemeinen Formel (Ib-17-Keto) mit einem Amin der Formel H-W-X-Y-Z-E , 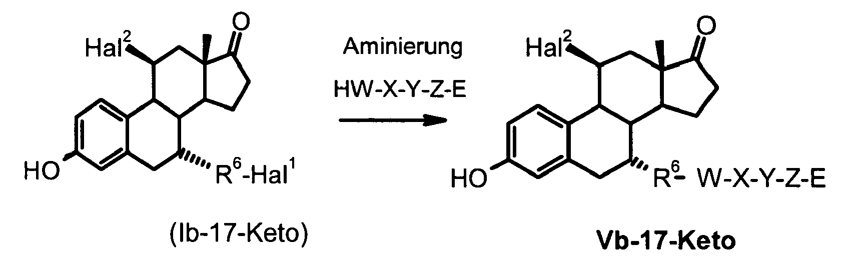
c) nukleophile Alkylierung der Position 17 der Verbindungen der Formel (Vb-17-Keto) unter Erhalt von Verbindungen der Formel (Vb) 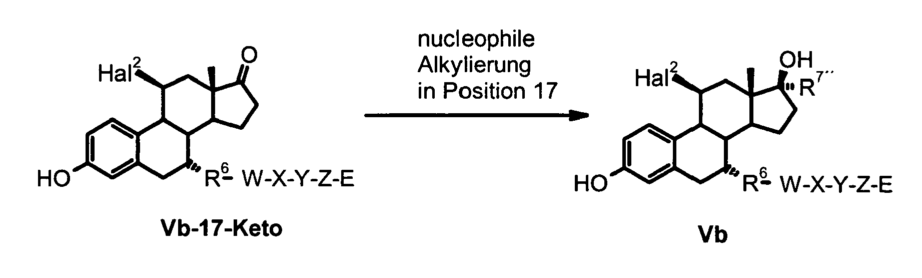
wobei Hal², R⁶-Hal¹ und R^{7"} die Bedeutungen gemäß Anspruch 3 besitzen und
W für -N(R⁷)- steht, wobei R⁷ Wasserstoff oder ein C₁-C₄-Alkylrest ist,
X für -(CH₂)_{q}- steht, wobei q = 0 oder eine ganze Zahl von 1 - 12 ist,
Y eine direkte Bindung zwischen X und Z oder eine -SOₙ-Gruppe ist, wobei n = 0,1 oder 2 ist,
Z ein gerad- oder verzweigtkettiger C₁-C₇Alkylenrest ist, der zumindest teilweise fluoriert ist,
E für -CF₃ oder für Pentafluorphenyl- steht.

9. Verfahren zur Herstellung von Verbindungen der allgemeinen Formel (Vb) mit den Teilschritten
a) Aromatisierung einer Verbindung der allgemeinen Formel (IIb-17-Keto) zu einer Verbindung der allgemeinen Formel (Ib-17-Keto) gemäß Anspruch 3 oder einem der Ansprüche 4 bis 7 in Abhängigkeit von Anspruch 3, 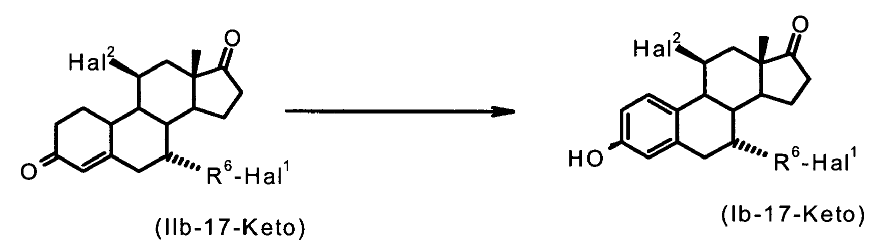
b) nukleophile Alkylierung der Position 17 der Verbindungen der Formel (Ib-17-Keto) unter Erhalt von Verbindungen der Formel (Ib-17β-OH) 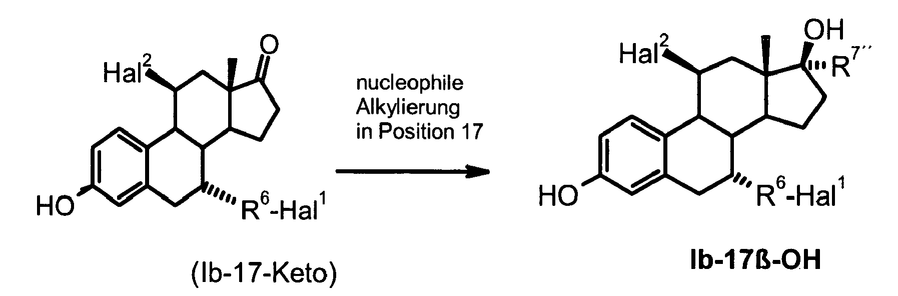
c) Umsetzung der Verbindungen der allgemeinen Formel (Ib-17-β-OH) mit einem Amin der Formel H-W-X-Y-Z-E unter Erhalt von Verbindungen der Formel (Vb), 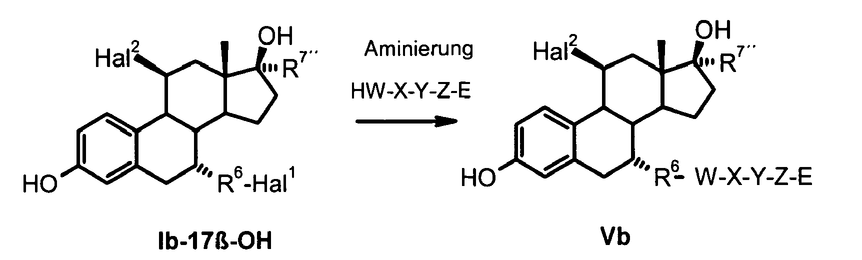
wobei Hal², R⁶-Hal¹ und R^{7"} die Bedeutungen gemäß Anspruch 3 besitzen und
W für -N(R⁷)- steht, wobei R⁷ Wasserstoff oder ein C₁-C₄-Alkylrest ist,
X für -(CH₂)_{q}- steht, wobei q = 0 oder eine ganze Zahl von 1 - 12 ist,
Y eine direkte Bindung zwischen X und Z oder eine -SOₙ-Gruppe ist, wobei n = 0,1 oder 2 ist,
Z ein gerad- oder verzweigtkettiger C₁-C₇Alkylenrest ist, der zumindest teilweise fluoriert ist,
E für -CF₃ oder für Pentafluorphenyl- steht.

10. Verfahren zur Herstellung von Verbindungen der allgemeinen Formel (Vc) mit den Teilschritten
a) Aromatisierung einer Verbindung der allgemeinen Formel (IIc-17-Keto) zu einer Verbindung der allgemeinen Formel (Ic-17-Keto) gemäß Anspruch 4 oder einem der Ansprüche 5 bis 7 in Abhängigkeit von Anspruch 4 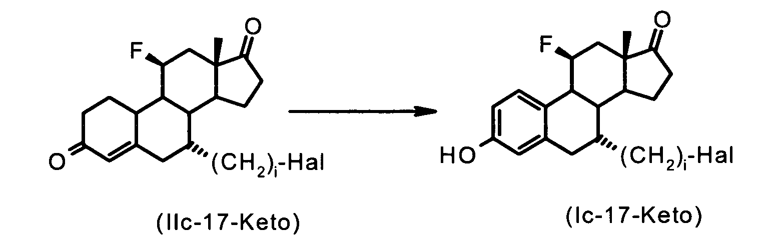
b) nachfolgende Umsetzung der Verbindungen der allgemeinen Formel (Ic-17-Keto) mit einem α-Alkyl(amin)-ω-perfluor(alkyl)-alkan der allgemeinen Formel (IV) zu einer Verbindung der allgemeinen Formel (Vc-Keto) 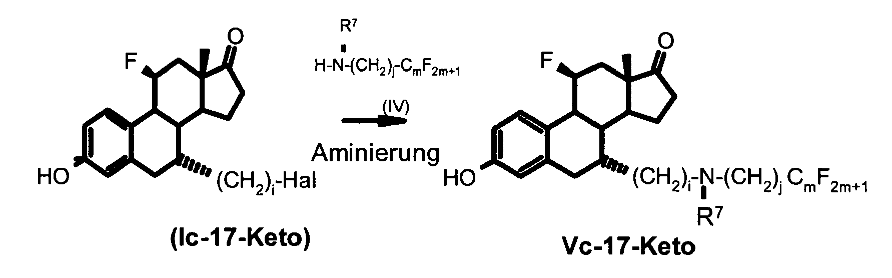
c) nucleophile Methylierung der Position 17 der Verbindungen der Formel (Vc-17-Keto) unter Erhalt von Verbindungen der Formel (Vc) 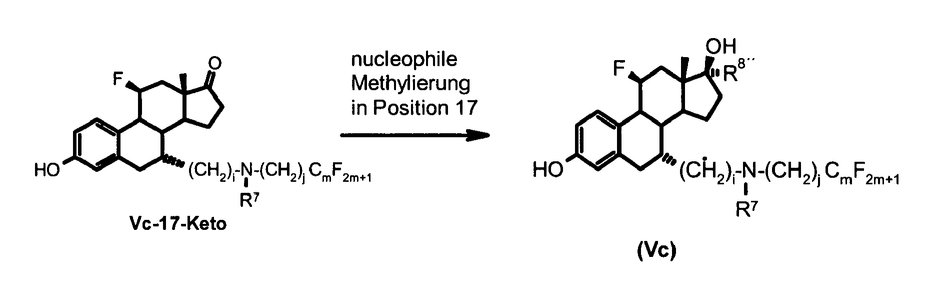
wobei
R^{8"} für eine Methylgruppe steht,
i für eine ganze Zahl von 3 bis 13 steht und
Hal für ein Chlor-, Brom- oder Iodatom steht.
R⁷ für einen C₁-C₄-Alkylrest steht,
j für eine ganze Zahl von 1 bis 10 steht und
m für eine ganze Zahl von 1 bis 5 steht.

11. Verfahren zur Herstellung von Verbindungen der allgemeinen Formel (Vc) mit den Teilschritten
a) Aromatisierung einer Verbindung der allgemeinen Formel (IIc-17-Keto) zu einer Verbindung der allgemeinen Formel (Ic-17-Keto) gemäß Anspruch 4 oder einem der Ansprüche 5 bis 7 in Abhängigkeit von Anspruch 4 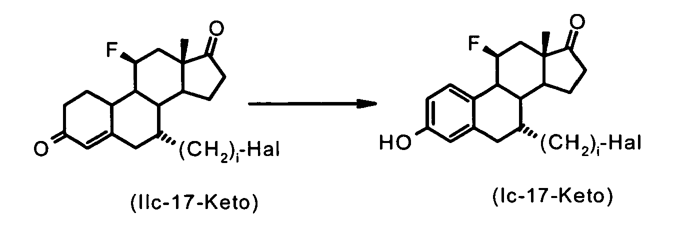
b) nachfolgende nucleophile Methylierung der Position 17 der Verbindungen der Formel (Ic-17-Keto) unter Erhalt von Verbindungen der Formel (Ic-17β-OH) 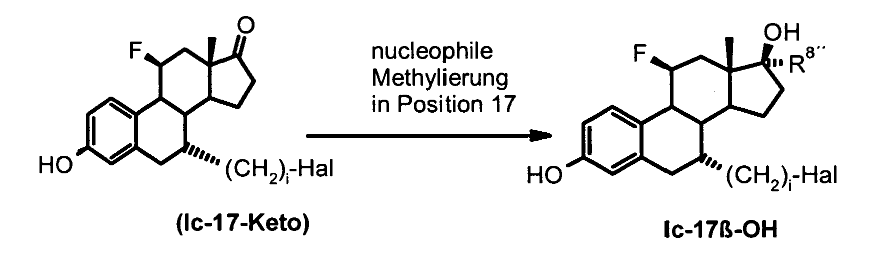
c) nachfolgende Umsetzung der Verbindungen der allgemeinen Formel (Ic-17-β-OH) mit einem α-Alkyl(amin)-ω-perfluor(alkyl)-alkan der allgemeinen Formel (IV) zu einer Verbindung der allgemeinen Formel (Vc) 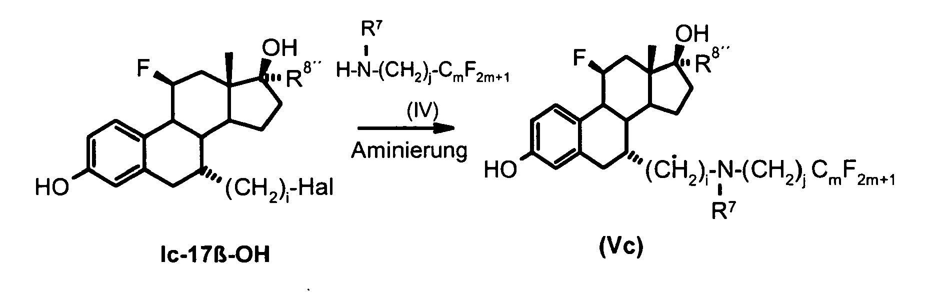
wobei
R^{8"} für eine Methylgruppe steht,
i für eine ganze Zahl von 3 bis 13 steht und
Hal für ein Chlor-, Brom- oder lodatom steht.
R⁷ für einen C₁-C₄-Alkylrest steht,
j für eine ganze Zahl von 1 bis 10 steht und
m für eine ganze Zahl von 1 bis 5 steht.
